(19) Europäisches Patentamt
European Patent Office
Office européen des brevets

(11) Veröffentlichungsnummer: **0 120 274 B1**

(12) # EUROPÄISCHE PATENTSCHRIFT

(45) Veröffentlichungstag der Patentschrift: **17.09.86**

(51) Int. Cl.⁴: **C 07 C 69/24,** A 23 L 1/226

(21) Anmeldenummer: **84101664.5**

(22) Anmeldetag: **17.02.84**

(54) **Neue 2-Methylpentansäureester, deren Herstellung und Verwendung als Riechstoffe sowie diese enthaltende Riechstoff-Kompositionen.**

(30) Priorität: **25.02.83 DE 3306560**

(43) Veröffentlichungstag der Anmeldung: **03.10.84 Patentblatt 84/40**

(45) Bekanntmachung des Hinweises auf die Patenterteilung: **17.09.86 Patentblatt 86/38**

(84) Benannte Vertragsstaaten: **CH DE FR GB LI NL**

(56) Entgegenhaltungen:
**GB - A - 1 306 017**
**US - A - 3 455 988**
**US - A - 3 966 986**

**CHEMICAL ABSTRACTS, NINTH COLLECTIVE INDEX, Bände 76-85, 1972-1976, Chemical Substances, Seite 27205CS, mittler Spalte, American Chemical Society, Columbus, Ohio, US;**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Blösl, Siegfried, Dr., Itterstrasse 33, D-4000 Düsseldorf 13 (DE)**
Erfinder: **Bruns, Klaus, Prof. Dr., Notburgaweg 6, D-4150 Krefeld-Traar (DE)**
Erfinder: **Schaper, Ulf Armin, Dr., Randstrasse 18a, D-4150 Krefeld (DE)**

**EP 0 120 274 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es wurde gefunden, dass 2-Methylpentansäureester der Formel

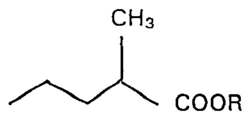

worin R ein ungesättigter, gerad- oder verzweigtkettiger oder carbocyclischer Kohlenwasserstoffrest mit 3 - 9 C-Atomen ist, wertvolle Riechstoffe mit kräftigen, meist vegetabilischen Geruchsnoten und hervorragender Haftfestigkeit sind.

Die Herstellung der Verbindungen erfolgt durch Veresterung von 2-Methylpentansäure mit einem ungesättigten, gerad- oder verzweigtkettigen oder carbocyclischen Alkohol oder Phenol entsprechend der vorstehenden allgemeinen Formel. Die Veresterungsreaktion erfolgt nach bekannten Verfahren der organischen Chemie.

In der angegebenen Formel kann R beispielsweise ein Propenyl-, Butenyl-, Hexenyl-, Nonenyl-, 3-Methyl-2-butenyl-, 3-Methyl-3-butenyl-, Cyclohexenyl-, 3,5,5-Trimethyl-2-cyclohexenyl-, (= Isophoryl-), 2,4(5)-Dimethyl-4-cyclohexenyl-methyl, Benzyl-, 3-Phenyl-2-propenyl-, Phenyl-, 2-Methylphenyl- oder 4-Ethylphenylrest sein. Besonders bevorzugt werden Reste mit 5 - 9 C-Atomen.

Eine bevorzugte Darstellung des 2-Methylpentansäure-3-methyl-2-butenylesters erfolgt durch Umsetzung von 2-Methylpentansäurechlorid mit 3-Methyl-2-buten-1-ol in Gegenwart von Pyridin. Auf diesem Wege können auch weitere der beanspruchten Ester hergestellt werden.

Die beanspruchten 2-Methylpentansäureester zeichnen sich durch interessante, sehr verschiedenartige, im wesentlichen vegetabilische Geruchsnoten aus. Vorzugsweise finden sich blumige und fruchtige Noten sowie Gerüche nach grünen oder getrockneten Pflanzenteilen. Mit abnehmender Kettenlänge, insbesondere unter C5, macht sich ein stechender Nebengeruch bemerkbar. Die Verbindungen besitzen eine aussergewöhnliche Haftfestigkeit und lassen sich sehr gut mit üblichen Parfüminhaltstoffen und anderen Riechstoffen zu neuartigen Kompositionen kombinieren, denen sie ebenfalls eine hohe Haftfestigkeit verleihen. Der Anteil der 2-Methylpentansäureester in den Riechstoffkompositionen beträgt im allgemeinen 1 - 50 Gewichtsprozent. Derartige Kompositionen können zur Parfümierung von kosmetischen Präparaten, wie Cremes, Lotionen, Duftwässern, Aerosolen, Tolietteseifen, Mundpflegemitteln usw., sowie in der Extrait-Parfümerie verwendet werden. Sie können ferner zur Geruchsverbesserung technischer Produkte, wie Wasch- und Reinigungsmittel oder Weichspüler, dienen. Es werden etwa 0,05 - 2 Gewichtsprozent einer derartigen Komposition, bezogen auf das gesamte Produkt, eingesetzt.

Aus DE-OS 2 530 227 sind niedere Alkylester der 2-Methylpentensäure, gegebenenfalls im Gemisch mit niederen Alkylestern der 2-Methylpentansäure bekannt, die als Würzessenz für flüssige und feste Lebensmittel und dergleichen bzw. als Parfüm für kosmetische und hygienische Präparate dienen. Es werden jedoch weder die beanspruchten 2-Methylpentansäureester mit ungesättigter- und/oder verzweigtkettiger oder carbocyclischer Alkoholkomponente, noch deren besondere Eigenschaften erwähnt.

*Beispiele*

A. *Allgemeine Herstellvorschrift*

0,1 Mol 2-Methylpentansäure, 0,12 Mol ungesättigter Alkohol oder Phenol, 100 ml Cyclohexan und 0,1 g para-Toluolsulfonsäure werden zum Sieden erhitzt. Das freigesetzte Wasser wird azeotrop ausgekreist. Nach beendeter Umsetzung wird das auf Raumtemperatur abgekühlte Reaktionsgemisch zunächst mit 10%iger Natronlauge, danach mit gesättigter Natriumsulfatlösung neutral gewaschen, über Natriumsulfat getrocknet, im Vakuum eingeengt und über eine 20 cm Füllkörperkolonne destilliert.

Ausbeute: 70 - 90% d. Th.

Folgende Ester wurden nach dieser allgemeinen Herstellungsvorschrift hergestellt:

1. 2-Methylpentansäure-3-phenyl-2-propylester

$Kp_{0,09}$ 99°C $N_D^{20}$ = 1,5163

$^1$H-NMR ($CDCl_3$) δ = 7,26 ppm (5H, m)
δ = 6,45 ppm (2H, m)
δ = 4,73 ppm (2H, d)

Geruch: Bittermandel-Note, grün

2. 2-Methylpentansäure-isophorylester

$Kp_{0,09}$ 87°C $N_D^{20}$ = 2,4572

$^1$H-NMR ($CDCl_3$) δ = 5,38 ppm (2H, m)
δ = 2,43 ppm (1H, m)

Geruch: Safran-, Tabak-, Johannisbrot-Note

3. 2-Methylpentansäure-3-hexenylester

$Kp_{21}$ 111°C $N_D^{20}$ = 1,4348

$^1$H-NMR ($CDCl_3$) δ = 5,5 ppm (2H, m)
δ = 4,1 ppm (2H, t)

Geruch: grün, Carbid-, Zwiebelnote

4. 2-Methylpentansäure-3-methyl-2-butenylester

0,15 Mol 2-Methylbuten-2-ol-1 wurden mit 0,1 Mol Pyridin vorgelegt und unter Rühren und Eiskühlung mit 0,1 Mol 2-Methylpentansäurechlorid innerhalb einer Stunde versetzt. Es wurde eine Stunde nachgerührt und dann in mit HCl angesäuertes Eiswasser gegossen.

Anschliessend wurde die organische Phase mit wässriger $Na_2CO_3$-Lösung gewaschen, getrocknet und fraktioniert destilliert.

Ausbeute: 71% d. Th. an

2-Methylpentansäure-3-methyl-2-butenylester

$Kp_{0,07}$ 38°C $N_D^{20} = 1,4370$

$^1$H-NMR ($CDCl_3$) $\delta = 5,33$ ppm (1H, m)
$\delta = 4,58$ ppm (2H, d)

Geruch: Holunderblüten, Juniperus, schwarze Johannisbeere

*B. Riechstoffkomposition (Fruchtige Holzbase)*

| | |
|---|---|
| 2-Methylpentansäure-3-methyl--2-butenylester | 200 Gew.-Teile |
| Boisambrene forte® (Henkel) | 200 Gew.-Teile |
| p-tert.-Butylcyclohexylacetat | 150 Gew.-Teile |
| Caryophyllenacetat | 100 Gew.-Teile |
| -Methyljonon | 70 Gew.-Teile |
| Linalool | 60 Gew.-Teile |
| Cistusöl (10%ig in DEP) | 50 Gew.-Teile |
| Galaxolid | 40 Gew.-Teile |
| Patchouliöl | 30 Gew.-Teile |
| Guajylacetat | 40 Gew.-Teile |
| Aldehyd C 12 (10%ig in DEP) | 20 Gew.-Teile |
| Aldehyd C 14 (10%ig in DEP) | 20 Gew.-Teile |
| Aldehyd C 16 (19%ig in DEP) | 20 Gew.-Teile |
| | 1000 Gew.-Teile |

(DEP = Diethylphthalat)

**Patentansprüche**

1. Neue 2-Methylpentansäureester der Formel

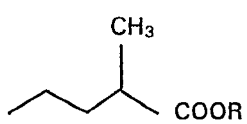

worin R ein ungesättigter, gerad- oder verzweigtkettiger oder carbocyclischer Kohlenwasserstoffrest mit 3 - 9 C-Atomen ist.

2. 2-Methylpentansäure-3-phenyl-2-propenylester.

3. 2-Methylpentansäure-isophorylester.

4. 2-Methylpentansäure-3-hexenylester.

5. 2-Methylpentansäure-3-methyl-2-butenylester.

6. Verfahren zur Herstellung von 2-Methylpentansäureestern des Anspruchs 1 durch Veresterung von 2-Methylpentansäure mit einem ungesättigten, gerad- oder verzweigtkettigen oder carbocyclischen Alkohol oder Phenol mit Resten R entsprechend der allgemeinen Formel.

7. Verfahren zur Herstellung von 2-Methylpentansäure-3-methyl-2-butenylester durch Umsetzung von 2-Methylpentansäurechlorid mit 3-Methyl-2-buten-1-ol in Gegenwart von Pyridin.

8. Verwendung von 2-Methylpentansäureestern der Ansprüche 1 - 5 als Riechstoffe.

9. Riechstoffkomposition, dadurch gekennzeichnet, dass sie 2-Methylpentansäureester gemäss Anspruch 1 - 5 in einer Menge von 1 - 50 Gewichtsprozent, bezogen auf die gesamte Komposition, enthalten.

**Claims**

1. New 2-methylpentanoic acid esters corresponding to the following formula

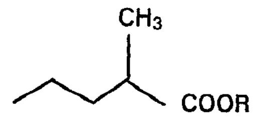

in which R is an unsaturated, straight-chain or branched-chain or carbocyclic hydrocarbon radical containing from 3 to 9 C-Atoms.

2. 2-Methylpentanoic acid-3-phenyl-2-propenyl ester.

3. 2-Methylpentanoic acid isophoryl ester.

4. 2-Methylpentanoic acid-3-hexenyl ester.

5. 2-Methylpentanoic acid-3-methyl-2-butenyl ester.

6. A process for the preparation of the 2-methylpentanoic acid esters claimed in claim 1 by esterification of 2-methylpentanoic acid with an unsaturated, straight-chain or branched-chain or carbocyclic alcohol or phenol containing radicals R corresponding to the general formula.

7. A process for the preparation of 2-methylpentanoic acid-3-methyl-2-butenyl ester by reaction of 2-methylpentanoic acid chloride with 3-methyl-2-buten-1-ol in the presence of pyridine.

8. The use of 2-methylpentanoic acid esters according to claims 1 to 5 as perfumes.

9. A perfume composition, characterized in that it contains 2-methylpentanoic acid esters according to claims 1 to 5 in a quantity of from 1 to 50% by weight, based on the composition as a whole.

**Revendications**

1. Esters de l'acide 2-méthylpentanoique nouveaux répondant à la formule

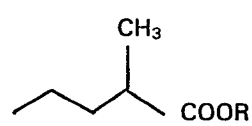

où R est un reste hydrocarbure à 3 à 9 atomes de C, insaturé, à chaîne droite ou ramifiée ou carbocyclique.

2. 3-phényl-2-propénylester de l'acide 2-méthylpentanoique.

3. Isophorylester de l'acide 2-méthylpentanoique.

4. 3-hexénylester de l'acide 2-méthylpentanoique.

5. 3-méthyl-2-buténylester de l'acide 2-méthylpentanoique.

6. Procédé pour la fabrication des ester de l'acide 2-méthylpentanoique, de la revendication 1, par estérification de l'acide 2-méthylpentanoique avec

un alcool ou un phénol insaturés, à chaîne droite ou ramifiée, ou carbocyclique, avec des restes R correspondant à la formule générale.

7. Procédé pour la fabrication du 3-méthyl-2--buténylester de l'acide 2-méthylpentanoique par réaction du chlorure d'acide 2-méthylpentanoique avec le 3-méthyl-2-butène-1-ol, en présence de pyridine.

8. Utilisation d'esters de l'acide 2-méthylpentanoique des revendications 1 à 5 comme substances odoriférantes.

9. Composition odoriférante caractérisé en ce qu'elle contient des esters de l'acide 2-methylpentanoique suivant les revendications 1 à 5, dans une proportion de 1 à 50% en poids calculé sur l'ensemble de la composition.